# EUROPEAN PATENT APPLICATION

(11) **EP 3 545 841 A1**
(43) Date of publication of application: **02.10.2019**
(21) Application number: 18460019.5
(22) Date of filing: 28.03.2018
(51) Int. Cl.: A61B 5/16, A61B 5/00, A61B 5/11, G16H 50/20

(54) **METHOD FOR EARLY DIAGNOSIS OF AUTISM SPECTRUM DISORDER IN CHILDREN**

(71) Applicant: Harimata Spolka Z O.O., 30-081 Krakow (PL)
(72) Inventor: Jarmolkowicz, Pawel, 11-040 Dobre Miasto (PL); Sobota, Krzysztof, 32-043 Skala (PL); Anzulewicz, Anna, 12-220 Ruciane Nida (PL)
(74) Representative: Karczmitowicz, Teresa Ewa

(57) **Abstract**

A method for early diagnosis of autism spectrum disorder (ASD) in a child, characterized in that four movement sensitivity parameteres are measured during an interaction of examined child with a touch screen, characterized in that:
i) Linear Movement Precision (LMP) is measured by calculating variation between a line drawn by ASD child and a line drawn by TD (typically developing) child, and wherein the numbers of knots used to draw both lines are calculated and compared;
ii) Polygonal Movement Precision (PMP) is measured by calculating the smallest area of a polygon drawn with knots generated by ASD child and of a polygon drawn with knots generated by TD child, and wherein these knots are connected with line sections and the smallest areas of resulting polygons are calculated and compared,
iii) Movement Acceleration Stability (MAS) is measured by calculating jerk defined as first derivative of acceleration while drawing a line by ASD child and while drawing a line by TD child, and wherein the jerk values are calculated and compared;
iv) Pressing Force Value (PFV) is measured so that the touch screen is placed on flat surface and force applied by ASD child or TD child while drawing a line is measured, and wherein the force values are calculated and compared.

## Description

Autism is a pervasive developmental disorder that has an impact on many different domains of a child's life. Proper diagnosis of autism spectrum disorder (ASD) requires more than good experience of a specialist medical practitioner or clinician based on all available evidence. The clinical picture of ASD is developed from three principal sources:
(i) the clinician's observations of the child;
(ii) the clinician's interviews with the child, it's parents, teachers, and/or caregivers; and
(iii) validated diagnostic instruments such as the Autism Diagnostic Observation Schedule (ADOS) and Autism Diagnostic Interview (ADI).

It has been recently proposed that abnormalities in the development of intentional movements are one of the early markers of ASD. These movement abnormalities are present before the manifestations of symptoms typically associated with autism, for example, deficiencies in social interaction and communication (Trevarthen and Delafield-Butt, 2013). A large range of motor symptoms have been documented throughout childhood, such as delays in the attainment of motor milestones (Teitelbaum et al., 1998), poor coordination (Ghaziuddin and Butler, 1998; Mari et al., 2003), unusual gait patterns (Fabbri-Destro et al., 2009; Matson et al., 2010; Ming et al., 2007; Schmitz et al., 2003) and difficulties in gross and fine motor skills (Lloyd et al., 2013). Motor development is crucial in a child's life because it impacts on the way the child interacts with the surrounding world and the human beings who are part of it. The means and manner of learning during the first months and years of life is determined by the skills and capacities the motor system affords (Adolph et al., 2010; Trevarthen, 1986, 1984) and is fundamental for the origin of thinking and future cognitive representation of the world.

Diagnosis of autism can be an intensive process and is subject to various errors. Most applications known to date use a mouse and a keyboard, but researchers have found that these solutions do not take into account the limited motor skills of autistic people. The tools to test for autism have not kept pace with the modern world so far. The proposed method removes that boundaries by using a tablet or other mobile device that incorporates multi-touch technology.

Two American solutions, below, use modern technologies. However, neither is clinically validated nor approved as a medical device for autism diagnosis. None of them has been approved as a "gold standard" in ASD diagnosis.

First solution is named *Akili* (http://www.brain.akiliinteractive.com/). Its aim is to create clinically validated cognitive therapeutic, assessment and diagnostic tools that look like video games. The first platform is Project EVO, a game supporting early assessment of autism (also: Alzheimer's disease, ADHD). It works on iPhones and iPads. This solution has received support from e.g. Autism Speaks, Pfizer, Shire Pharmaceuticals, or National Institute of Mental Health. It is an unfocused single game, the study results are not available.

Second solution is named *Cognoa* (https://www.cognoa.com/). The application is aimed to support the screening process for the ASD. It is designed to evaluate risk for developmental delay in children from 18 months to 7 years of age. The application is available for both iOS and Android. It offers two ways of tracking a child. First, parents can take free preliminary questionnaire to assess the child's risk of developmental disorders or delay. After using the questionnaire they can submit a question to the child development specialist. Second, parents can upload two short videos of child's natural behaviour at home that is viewed and assessed by Cognoa. The questionnaire takes five minutes to complete it. If they ask a question, they get the response within 3 days. Videos can be shorter than five minutes (sending of 2 videos is advised). An obvious advantage is that parents can submit a question to child development specialist, even when just using the questionnaire, with no need to go anywhere. They can have the child consulted without getting out of home. The method is however not considered a "gold standard" for ASD diagnosis. There is no evidence professional expertise is applied. Subjective evaluation is based on videos, which are not representative to the child's behaviour. The approach is not clinically validated and is not based on comparative analysis.

The current best validated sub-component of the "gold standard" in autism diagnosis, the ADOS Version 2, yields sensitivity and specificity values at most at 91% for sensitivity and 71% for specificity, or alternatively 82% for sensitivity and 88% for specificity when the algorithm is adjusted to increase specificity (de Bildt et al., 2009).

Korean patent KR101784429 (B1) relates to an early diagnosing system of autism and, more specifically, to a system for early diagnosis of autism and rehabilitation treatment of early autism. The invention provides a verification process to general children in the type of a game having amusement and interest to remotely diagnose coordination power of a sense of sight, a sense of perception, and thought of each child, thereby enabling an early autism diagnosis of each child and rehabilitation treatment in accordance with an autism diagnosis. A smart device is based on a screening value of the visual acuity, especially, by activating autism screening application using 3D character based on 3D map. It is possible to encourage enrolled children to voluntarily participate in the autistic screening process by encouraging the amusement and interest of registered children. The autistic examination application displays a 3D character on a touch screen provided in a smart device, and repeatedly performs a simple screening process in which a registered child touches a character displayed with a finger for a set touch time. The co-ordination value of the registered child is measured.

American patent application US2015282752 (A1) discloses a game board and method for diagnosing and treating neurological disorders such as attention deficits and/or comorbidities, attention deficit hyperactivity disorder, and/or conditions with attentional impairment such as autism spectrum disorder, anxiety, depression, and epilepsy. The game board has two sets of marks, each set forming a line, wherein a first line is orientated in a first direction parallel with a side of the rectangular game board, and a second line is orientated in a second direction diagonal to the first line. Objective cubes are variously positioned on the marks and the player is instructed to hit an objective cube according to whether it displays a particular image. Distractors are employed to assist in diagnosing and treating the disorder. Once the game (e.g.16 sessions) is complete, the data recorded regarding player performance, which includes movement, reaction, correct, incorrect, and omissions is wirelessly transmitted to the cloud for analysis. The applicants have developed algorithms to make sense of this information, c.f. US9014614 (B2). The response differences between spatial and non-spatial distractions is a key element in understanding conditions associated with attention like ADHD, anxiety, depression, and autism.

In the above mentioned patent document US9014614 (B2) a cognitive disorder diagnostic and treatment system that employs cognitive cubes, gameplay associated with the cognitive cubes, and a data gathering as statistical analysis base device that may be a computer, that communicates the gathered data to a web server host according to a unique ID associated with particular cognitive cubes and further associated with a particular player are disclosed. Using the statistical data gathered using the gameplay, various cognitive disorders may be successfully diagnosed and treated with higher reliability.

Another American patent application US2012323084 (A1) describes a set of blocks, for playing a game. Each block has one characteristic selected from the first category and one characteristic selected from the second category. At least some of the characteristics in the first category are different from one another and at least some of the characteristics in the second category are different from one another. Categories may include shape, size, colour, texture, weight and surface finish. The blocks can be provided with a game board and a die, for playing a game or for diagnosis. The blocks can be used for play, and for diagnosis of children with autism or other individuals with other neurological disorders. The child is instructed to sort the blocks according to the selected cathegory, or unspecified cathegory comprising a sort criterion. The child is provided with feedback as to whether blocks are sorted correctly or not.

Movement abnormalities are a core feature of autism, but to date they are rarely used in diagnostic assessments (e.g. ADOS, ADI-R). However, the quickly developing technology makes new possibilities in diagnosis of autism. New and relatively cheap inertial and gyroscopic sensors, or screen-based touch sensors are now available in most of the mobile devices like smartphones or tablets. These are an untapped resource that could greatly improve sensitivity and specificity in autism diagnosis.

According to the present invention, new method for autism diagnosing combines attractive gameplay on a mobile device with assessment of a child's motor interaction with the touchscreen. Current pilot trials demonstrate a sensitivity and specificity of the mobile device-based autism assessment at best at 83% for sensitivity and as much as 85% for specificity (Anzulewicz, Sobota & Delafield-Butt, 2016). The proposed solution is attractive and timely. The children enjoy the gameplay, no special training is required for its implementation, and it potentially yields results equivalent to - or even better than - the current "gold standard" in autism diagnostic assessment. It exploits rapid technological advances in commercial motion sensor development now deployed in the mobile device enabling diagnosis of autism.

The proposed method is designed as a series of educational applications for a tablet or other mobile device. They are specially adjusted for the diagnosis of developmental abnormalities in children of age of 2-5 years. The solution combines two interactive games with behavioural psychology and big data methods to collect up to 272 indicators per second during the gameplay. Proprietary data mining and machine learning algorithms allow detection of signs of ASD based on analysis of individual behavioural patterns, e.g. touchscreen dynamics or reactions to game flow. New data is compared to reference data collected from both typically developing (TD) children, and those with confirmed ASD. The result is a unique to the child comprehensive functional behaviour profile across multiple domains. Resulting profile can be available on the mobile device and/or may be sent as a PDF report, e.g. in an email attachment.

Two games named below "Sharing" and "Creativity" are the main compounds of the proposed method. The whole procedure includes the following steps:
1. Fill in the user's data: login, sex, age.
2. Training before first stage - 2 minutes.
3. First stage - "Sharing" - 5 minutes.
4. Training before second stage - 2 minutes.
5. Second stage - "Creativity" - 5 minutes.
6. Sending data to the server.
7. Computations: pre-processing, machine learning, generation of results.
8. Generation of basic report.

Training before first and second stages (games) is necessary for the child to learn how the gameplay should be conducted. Training is run with an assistance of an adult explaining what should be done.

Both first and second stages ("Sharing" and "Creativity" games) measure mainly precision and stability of movement. The following parameters are measured:
A. Linear Movement Precision (LMP) is measured by calculating variation between a line drawn by ASD child and a line, usually straight line, drawn by TD child. Number of points (knots) used to draw the line is calculated. ASD children usually require more knots to complete the task, therefore the assessment is based on bigger number of knots as the patterns are more curved.
B. Polygonal Movement Precision (PMP) is also measured by calculating the smallest area of a polygon drawn with points (knots) generated by a child. The child is drawing several points when interacting with the touchscreen. ASD child is usually making more chaotic patterns with boundary points scattered on the screen. These points are then connected and the smallest area is calculated. The trial tests proved that for ASD children this area is larger (i.e. the knots are more scattered) than for TD children.
C. Movement Acceleration Stability (MAS) is measured by calculating jerk (first derivative of acceleration or second derivative of speed), which provides valuable information especially about beginning and finishing phases of the movement. TD children are touching the screen lightly when starting the movement and finish it smoothly. Conversely, ASD children are making sharp, jerky movements, which can thus be quantified by jerk.
D. Pressing Force Value (PFV). The three parameters described above use an interaction between the child and the touchscreen. The last parameter is related to the accelerometer and gyroscope. The tablet/mobile device shall be placed on flat surface e.g. the table and the child shall not keep it in hands. When the screen is pressed, the force applied can be assessed by calculating the movement along the axis perpendicular to the screen. The clinical observations show that ASD children are using significantly more force when pressing the screen.

The game "Sharing" is about distributing food, e.g. fruits equally between four cartoon characters. The game consists of a series of sharing trials, where a new trial means distribution of new portion of food. The child's task is identical in each trial, however the food item, which the child has to divide and share, differs from trial to trial. The food can be easily divided into four even rations with a simple gesture, each ration can then be dragged and dropped onto the plate in front of each cartoon character. If the child proceeded like this he/she will be granted with a positive feedback in the form of nice facial expression (smile) of the cartoon character.

The visual and audio screen is supplemented with a number of distractors which were proven to constitute an important part of the game. Exemplary distractors are singing bird, rotating fan, banana, yellow duck, lamps which can be switched on/off and others. The distractors are moving, emitting sounds and are interactive. It has been noted that ASD children are much more susceptible to be distracted than TD children.

The game "Creativity" is about outlining and colouring an easy shape on the screen. The child first chooses the shape he/she wants to play with, then outlines it step-by-step with his/her finger, and finally colours it using a colour palette. During the game the algorithms measure precision and stability of the movements as described earlier.

The results of the "Creativity" game contribute significantly to the specificity, which is the measure of children without ASD correctly identified as healthy with the invented method.

The proposed method is illustrated on the drawing, where:

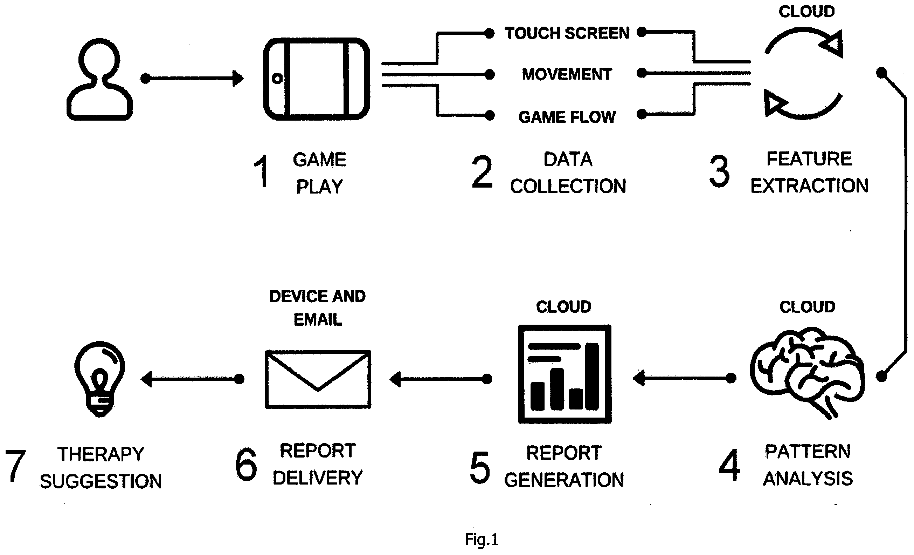

Fig. 1 shows general idea of the methodology;

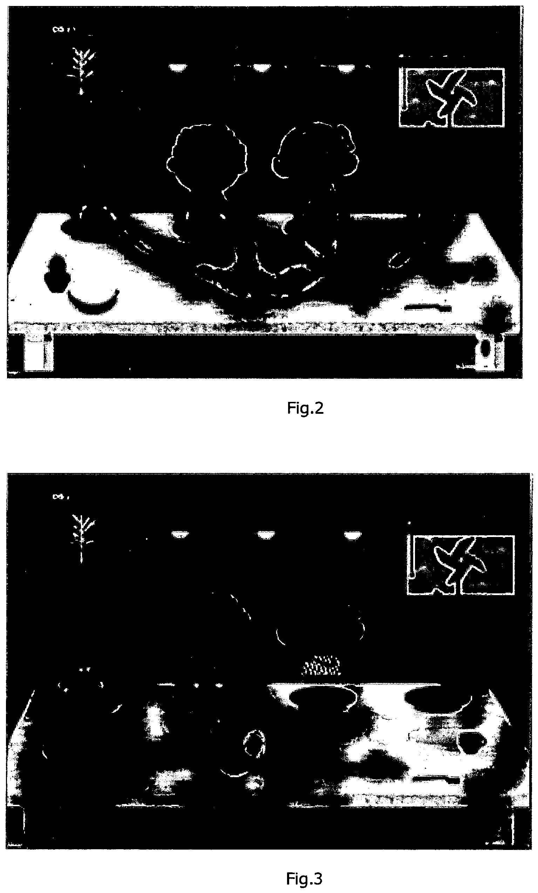

Fig. 2 shows first exemplary heat map for the game "Sharing" - for even sharing;
Fig. 3 shows second exemplary heat map for the game "Sharing" - for uneven sharing;

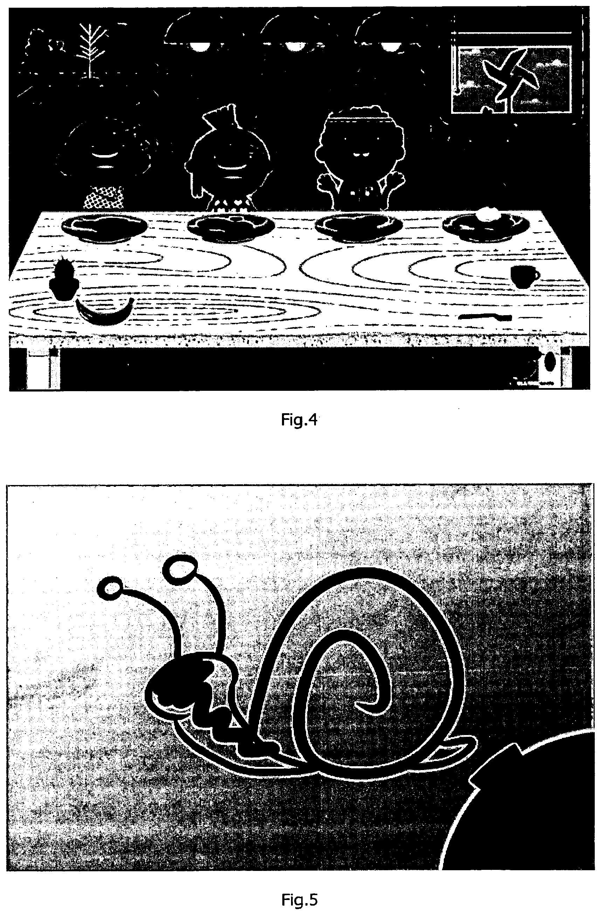

Fig. 4 shows positive feedback if the food has been shared evenly;
Fig. 5 shows exemplary screen in "Creativity" game; and

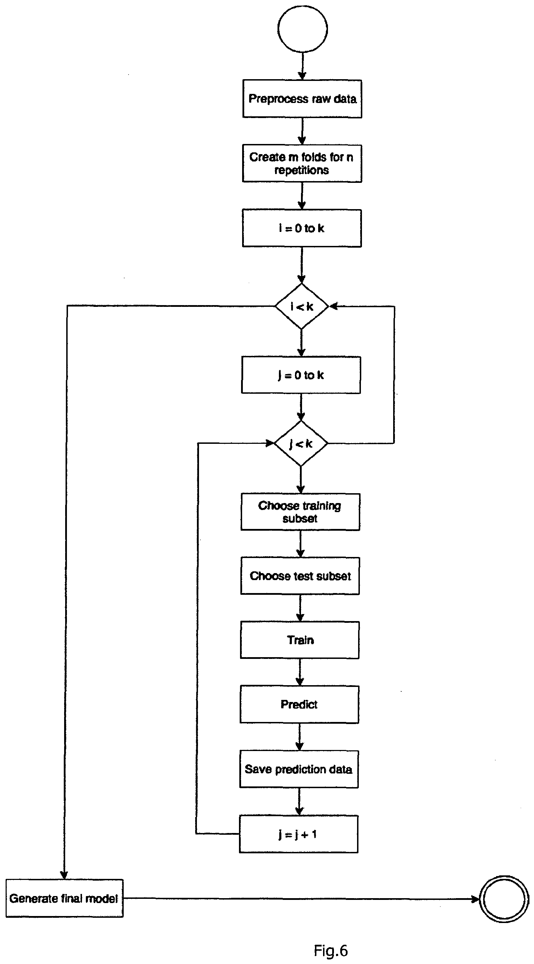

Fig. 6 shows a diagram of the modeling algorithm.

The machine learning approach is used to process data obtained from the tablet sensors as well as basic information about the child (age, sex). The model calculates the probability that a particular child's data belong to the ASD group or the control one (TD).

Parameters: 272 features were extracted from the device data to give a comprehensive computational description of the child's movements sensed by the device, and made in interaction with it. These features were obtained from the Screen (108 features) and Inertial (164 features) data. Of these features, 26 were highly correlated (r > 0.9), considered redundant, and reduced to a single feature. 247 features from device sensors and touch, together with information about child's age and gender, were included in the final analysis.

Goal function: The fitting of the machine learning algorithm is conducted by solving an optimisation problem, which is to minimise the error between the clinical diagnosis (ASD or not) and the results obtained with the proposed method (probability of having autism) by changing fitting parameters.

Building the model: To build models able to predict group classification (ASD or control TD), and to ensure reliable classification, a k-fold cross validation procedure was employed. This method was used to establish the predictive power of the model, i.e. how the result ought to generalise to an independent dataset. To increase the stability of the result, additional k repetitions of the process were performed. The full dataset of calculated features was split into k equal sized samples. From k subsamples one was chosen for the validation (test), and the rest (k-1) were used as the training dataset. Every sample was used exactly once as a validation (test) dataset. This process was performed k times (folds). During every iteration model was trained on the k-1 sample and then tested on the one sample left for the prediction. Results of the prediction were stored, to later establish the end-point model, which combines the results from each fold. This process was repeated k times. In the end k × k samples were created and tested. Based on the prediction data gathered during both iterations (repetitions and folds), the Receiver Operating Characteristics (ROC) curve was generated, and the sensitivity (true positive rate) and specificity (true negative rate) were calculated.

The outcome of the algorithm is the probability that the child shows signs characteristic of autism, defined within range of 0-100%. Result above 50% indicates that the child exhibits early symptoms of autism.

### References:

Adolph, K.E., Tamis-Lemonda, C.S., Karasik, L.B., 2010. Cinderella indeed - a commentary on Iverson's "Developing language in a developing body: the relationship between motor development and language development". Journal of Child Language 37, 269-273. doi:10.1017/S030500090999047X
Anzulewicz, A., Sobota, K., Delafield-Butt, J. T., 2016. Toward the autism motor signature: Gesture patterns during smart tablet gameplay identify children with autism. Scientific Reports, 6. doi:10.1038/srep31107
de Bildt, A., Sytema, S., van Lang, N.D.J., Minderaa, R.B., van Engeland, H., de Jonge, M.V., 2009. Evaluation of the ADOS Revised Algorithm: The Applicability in 558 Dutch Children and Adolescents. J Autism Dev Disord 39, 1350-1358. doi:10.1007/s10803-009-0749-9
Fabbri-Destro, M., Cattaneo, L., Boria, S., Rizzolatti, G., 2009. Planning actions in autism. Exp Brain Res 192, 521-525. doi:10.1007/s00221-008-1578-3
Ghaziuddin, M., Butler, E., 1998. Clumsiness in autism and Asperger syndrome: a further report. J Intellect Disabil Res 42 (Pt 1), 43-48.
Kanner, L., 1968. Autistic disturbances of affective contact. Acta Paedopsychiatr 35, 100-136.
Mari, M., Castiello, U., Marks, D., Marraffa, C., Prior, M., 2003. The reach-to-grasp movement in children with autism spectrum disorder. Philosophical Transactions of the Royal Society of London B: Biological Sciences 358, 393-403. doi:10.1098/rstb.2002.1205
Matson, J.L., Mahan, S., Kozlowski, A.M., Shoemaker, M., 2010. Developmental milestones in toddlers with autistic disorder, pervasive developmental disorder--not otherwise specified and atypical development. Dev Neurorehabil 13, 239-247. doi:10.3109/17518423.2010.481299
Ming, X., Brimacombe, M., Wagner, G.C., 2007. Prevalence of motor impairment in autism spectrum disorders. Brain and Development 29, 565-570. doi:10.1016/j.braindev.2007.03.002
Schmitz, C., Martineau, J., Barthélémy, C., Assaiante, C., 2003. Motor control and children with autism: deficit of anticipatory function? Neuroscience Letters 348, 17-20. doi:10.1016/S0304-3940(03)00644-X
Teitelbaum, P., Teitelbaum, O., Nye, J., Fryman, J., Maurer, R.G., 1998. Movement analysis in infancy may be useful for early diagnosis of autism. PNAS 95, 13982-13987. doi:10.1073/pnas.95.23.13982
Trevarthen, C., 1986. Development of intersubjective motor control in infants., in: Motor Development in Children: Aspects of Coordination and Control. Martinus Nijhoff, Dordrecht, pp. 209-261.
Trevarthen, C., 1984. How Control of Movement Develops, in: Human Motor Actions: Bernstein Reassessed. Elsevier, Amsterdam, pp. 223-261.
Trevarthen, C., Delafield-Butt, J.T., 2013. Autism as a developmental disorder in intentional movement and affective engagement. Front. Integr. Neurosci. 7. doi:10.3389/fnint.2013.00049

## Claims

1. A method for early diagnosis of autism spectrum disorder (ASD) in a child, **characterized in that** four movement sensitivity parameteres are measured during an interaction of examined child with a touch screen, wherein:
i) Linear Movement Precision (LMP) is measured by calculating variation between a line drawn by ASD child and a line drawn by TD (typically developing) child, and wherein the numbers of knots used to draw both lines are calculated and compared;
ii) Polygonal Movement Precision (PMP) is measured by calculating the smallest area of a polygon drawn with knots generated by ASD child and of a polygon drawn with knots generated by TD child, and wherein these knots are connected with line sections and the smallest areas of resulting polygons are calculated and compared,
iii) Movement Acceleration Stability (MAS) is measured by calculating jerk defined as first derivative of acceleration while drawing a line by ASD child and while drawing a line by TD child, and wherein the jerk values are calculated and compared;
iv) Pressing Force Value (PFV) is measured so that the touch screen is placed on flat surface and force applied by ASD child or TD child while drawing a line is measured, and wherein the force values are calculated and compared.

2. A method according to claim 1, **characterized in that** a mobile device is applied and **in that** the touch screen is the one of a mobile device.

3. A method according to claim 2, **characterized in that** the mobile device is a tablet or a smartphone.

4. A method according to claim 1 or 2, or 3, **characterized in that** it comprises a procedure of assessing a sharing ability of the child which is distributing food between four cartoon characters on the touch screen, and wherein the procedure consists of a series of sharing trials, where every new trial means distribution of new portion of food, and wherein the child's task is identical in each trial, however the food item, which the child has to divide and share, differs from trial to trial, and wherein food can be easily divided into four even rations with a simple gesture, each ration can then be dragged and dropped onto the plate in front of each cartoon character which then makes a positive feedback to the child if provided with the food ration.

5. A method according to claim 1 or 2, or 3, or 4, **characterized in that** it comprises a procedure of assessing a creativity ability of a child which is instructed to outline and colour a shape on the touch screen, and wherein the child first chooses the shape he/she wants to draw, then outlines it step-by-step with his/her finger, and finally colours it using a colour palette, and wherein during the procedure precision and stability of the movements are measured.

6. A method according to claim 1 or 2, or 3, or 4, or 5, **characterized in that** the visual and/or audio screen are supplemented with one or more distractors which are moving, emitting sounds and/or are interactive in course of the procedure, and thus interfere with what is displayed on the touch screen and/or made audible to examined child.
